# FASCICULE DE BREVET EUROPEEN

(11) **EP 2 413 891 B1**
(45) Date de publication et mention de la délivrance du brevet: **21.09.2016**
(21) Numéro de dépôt: 10715914.7
(22) Date de dépôt: 01.04.2010
(51) Int. Cl.: A61K 8/64, C07K 5/08, C07K 7/06, A61K 38/06, A61K 38/08

(54) **PEPTIDES ANTI-AGE ACTIVATEURS DU PROTEASOME ET COMPOSITIONS LES CONTENANT**
GEGEN DIE ALTERUNG GERICHTETE PEPTIDE, DIE DAS PROTEASOME AKTIVIEREN, UND SIE ENTHALTENDE ZUSAMMENSETZUNGEN
ANTI AGING PEPTIDES WHICH ACITIVATE THE PROTEASOME, AND COMPOSTIONS COMPRISING THEM

(30) Priorité: 02.04.2009 FR 0901612
(43) Date de publication de la demande: 08.02.2012
(73) Titulaire: ISP Investments Inc., Wilmington, DE 19805 (US)
(72) Inventeur: DAL FARRA, Claude, 12446 Kerhonkson New York (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2010/000279
(87) Numéro de publication internationale: WO 2010/112712

(56) Documents cités:
- WO-A1-2005/061530

### SEQUENCE LISTING

<110> ISP Investments Inc
<120> NOUVEAUX PEPTIDES ANTI-AGE ACTIVATEURS DU PROTEASOME ET COMPOSITIONS LES CONTENANT
<130> Bv 09-120
<150> FR 09 01612
   <151> 2009-04-02
<160> 6
<170> PatentIn version 3.3
<210> 1
   <211> 4
   <212> PRT
   <213> Artificial
<220>
   <223> synthetic peptide
<220>
   <221> MOD_RES
   <222> (4)..(4)
   <223> AMIDATION
<400> 1
   Ile Arg Lys Gly
   1
<210> 2
   <211> 5
   <212> PRT
   <213> Artificial
<220>
   <223> Synthetic peptide
<220>
   <221> MOD_RES
   <222> (5)..(5)
   <223> AMIDATION
<400> 2.
   Leu Arg Lys Gly Met
   1               5
<210> 3
   <211> 5
   <212> PRT
   <213> Artificial
<220>
   <223> Synthetic peptide
<220>
   <221> MOD_RES
   <222> (5)..(5)
   <223> AMIDATION
<400> 3
   Pro Arg Lys Gly Thr
   1               5
<210> 4
   <211> 5
   <212> PRT
   <213> Artificial
<220>
   <223> Synthetic peptide
<220>
   <221> MOD_RES
   <222> (5)..(5)
   <223> AMIDATION
<400> 4
   Val Arg Lys Gly Phe
   1               5
<210> 5
   <211> 5
   <212> PRT
   <213> Artificial
<220>
   <223> synthetic peptide
<220>
   <221> MOD_RES
   <222> (5)..(5)
   <223> AMIDATION
<400> 5
   Val Arg Lys Gly Tyr
   1               5
<210> 6
   <211> 3
   <212> PRT
   <213> Artificial
<220>
   <223> synthetic peptide
<220>
   <221> MOD_RES
   <222> (3)..(3)
   <223> AMIDATION
<400> 6
   Arg Lys Gly
   1

## Description

La présente invention se rapporte à des composés peptidiques de formule générale R₁ - X₁ - Arg - Lys - Gly - X₂ - R₂ ainsi que leurs utilisations en cosmétique et/ou, pharmaceutique afin de prévenir et/ou corriger les effets du vieillissement et du photo-vieillissement de la peau et des phanères, et protéger la peau contre les agressions dues aux rayonnements UV.

Le vieillissement correspond à l'ensemble des processus physiologiques et psychologiques qui modifient la structure et les fonctions de l'organisme à partir d'un certain âge. On distingue deux types de vieillissement, d'une part, le vieillissement intrinsèque et, d'autre part, le vieillissement extrinsèque. Le vieillissement intrinsèque est du à des facteurs génétiques, à des modifications biochimiques qui ont lieu lors d'états de fatigue, de stress, de changements hormonaux comme lors de la grossesse... Le vieillissement extrinsèque, quant à lui, est dû à des facteurs environnementaux auxquels est soumis l'organisme tout au long de sa vie, tels que la pollution, le soleil, les maladies etc. Il s'agit d'un processus lent et progressif qui atteint toutes les cellules de l'organisme par différents moyens et se manifeste de différentes manières. Par exemple, au niveau de la peau, l'aspect de celle-ci est modifié par les divers types d'agressions internes ou externes et l'on voit apparaître alors des rides et ridules, des taches d'hyper ou d'hypo-pigmentation, une sècheresse voire une déshydratation de la peau, un amincissement de l'épiderme, une élastose, des imperfections, des taches de vieillesse.... Tous ces changements affectent non seulement la peau, mais également les phanères tels que les ongles et les cheveux. Ces modifications sont dues entre autres à l'altération des fonctions de renouvellement cellulaire, de cohésion cellulaire, de synthèse de collagène, d'élastine et d'autres protéines, et conduisent finalement à une diminution des qualités de barrière protectrice de la peau et à un aspect peu esthétique de celle-ci. Mais l'un des processus principaux responsables du vieillissement des cellules est sans conteste l'accumulation de protéines endommagées dans ces dernières. En effet, les protéines sont la cible de diverses modifications post-traductionnelles anormales telles que l'oxydation, la glycation, la conjugaison avec des produits issus de la peroxydation lipidique, phénomènes dont l'incidence augmente fortement avec l'âge.

Il est connu que les radicaux libres jouent un rôle clé dans le processus de vieillissement et plus particulièrement dans la formation de protéines oxydées, endommagées (Harman et al. «Aging: a theory based on free radical and radiation chemistry » J. Gerontol., 11, 298-300). L'accumulation de protéines endommagées pose donc le problème de l'efficacité des systèmes protéolytiques en charge de l'élimination de ces protéines, et particulièrement celle du système protéosomal impliqué non seulement dans l'élimination des protéines altérées, notamment par oxydation, mais aussi dans le renouvellement continu des protéines intracellulaires.

La voie ubiquitine-protéasome joue un rôle fondamental dans un très grand nombre de processus biologiques. En effet, les mécanismes de dégradation des protéines par le protéasome sont impliqués dans des mécanismes cellulaires importants tels que la réparation de l'ADN, le contrôle de l'expression des gènes, la régulation de la progression du cycle cellulaire, le contrôle de la qualité des protéines néo-synthétisées, l'apoptose ou la réponse immunitaire (Glickman and Ciechanover, 2002).

Le protéasome présent dans les cellules humaines est un complexe multi protéique de très grande taille présent dans le cytoplasme et le noyau. Les formes purifiées du protéasome comprennent 2 grandes sous-unités ; d'une part, un coeur protéolytique appelé protéasome 2OS et, d'autre part, un complexe régulateur 19S qui se lie à chacune des deux extrémités du protéasome 2OS (Coux et *al*., 1996 ; Glickman et Coux, 2001). Le protéasome 2OS est une particule en forme de cylindre creux, composée de 28 sous-unités alpha et béta, distribuées en 4 anneaux heptamériques. Les activités peptidases sont présentes à la surface interne du cylindre et s'influencent de manière allostérique. Trois activités protéolytiques (« trypsine, chymotrypsine et caspase-like ») ont été associées au protéasome 2OS et concourent à la destruction des protéines en peptides inactifs de 3 à 20 acides aminés. Outre le protéasome 2OS, le protéasome 26S comprend le complexe régulateur 19S de 0,7 MDa constitué de 20 sous-unités environ. Des études récentes d'immunopurification ont montré que d'autres protéines pouvaient être associées au protéasome 20S et 19S (par exemple le complexe régulateur 11S).

Au vu de la diversité des processus cellulaires contrôlés via la dégradation des protéines, il n'est pas surprenant de constater que des altérations de la voie ubiquitine-protéasome sont à l'origine, ou étroitement liées à plusieurs maladies génétiques et à de nombreuses pathologies humaines telles que les cancers colorectaux, les lymphomes, les syndromes inflammatoires, les maladies neuro-dégénératives telles que Parkinson ou Alzheimer....

Des travaux menés ces dernières années ont permis de corréler le vieillissement avec l'activité du protéasome. En effet, alors qu'avec l'âge il y a une augmentation de l'accumulation des protéines oxydées, on a constaté une baisse de l'efficacité du système protéosomal (Petropoulos et al., J. Gerontol. A. Biol. Sci. 2000, 55A :B220-7). Cette baisse de l'efficacité du système protéosomal était due en fait à une baisse de la quantité de protéasome. Ces résultats ont été confirmés par ceux d'une étude portant sur la quantité et l'activité du protéasome dans des cellules d'individus centenaires versus individus jeunes (Chondrogianni et al., Exp. Gerontol. 2000 ;35 :721-8). Ces études, ainsi que bien d'autres, démontrent bien le lien entre vieillissement et activité du protéasome et l'on peut penser que l'induction de l'expression du protéasome dans des cellules de la peau ou des phanères pourrait avoir une influence positive sur le vieillissement, voire retarder celui-ci.

Dans l'optique de prévenir ou retarder le vieillissement, des compositions cosmétiques « naturelles » ont été proposées: par exemple le brevet FR 2822701 divulgue une composition à base d'un extrait d'algue phaeodactylum pour favoriser l'activité du protéasome. Ou encore, la demande de brevet FR 2898808 décrit l'utilisation d'une composition comprenant un extrait de micro-algue et de ferrulate d'arginine, toujours pour activer le protéasome. D'autres compositions quant à elle comprennent des composés chimiques, capables de moduler l'activité du protéasome pour avoir un effet antivieillissement. Ces compositions sont décrites dans les demandes de brevets WO 2006/105811 ou encore WO 2005/061530. Cependant les composés de type peptidiques proposés présentent une taille importante et il est difficile dans le domaine de la cosmétique de les utiliser tels quels. Il existe alors un besoin, notamment dans l'industrie cosmétique, de nouveaux composés présentant une taille réduite et efficace quant à leur utilisation en tant qu'activateur du protéasome.

C'est ainsi que la Demanderesse a découvert que des composés de formule R₁ - X₁ - Arg - Lys - Gly - X₂ - R₂. étaient capables d'activer le protéasome et pouvaient ainsi être utiles afin de prévenir ou traiter les signes cutanés du vieillissement ainsi que les agressions dues aux rayonnements UV.

Par conséquent la présente invention a pour premier objet un composé peptidique de formule générale suivante (I) :

R₁ - X₁ - Arg - Lys - Gly - X₂ - R₂.

Dans laquelle,
X1 représente une isoleucine, une leucine, une proline, une valine, ou est égal à zéro,
X2 représente une cystéine, un acide glutamique, une glutamine, une histidine, une méthionine, une phénylalanine, une tyrosine, une thréonine, ou est égal à zéro,
R1 représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R2 représente le groupe hydroxyle de la fonction carboxyle de l'acide aminé C-terminal, substitué par un groupement protecteur pouvant être choisi parmi une chaîne alkyle de C1 à C20, ou un groupement NH2, NHY ou NYY avec Y représentant une chaîne alkyle de C1 à C4,
ladite séquence de formule générale (I) étant constituée de 3 à 5 résidus d'acides aminés,
ladite séquence de formule générale (I) pouvant comprendre des substitutions des acides aminés X1 et X2, par d'autres acides aminés chimiquement équivalents.

La présente invention a pour second objet une composition cosmétique comprenant à titre de principe actif ledit composé peptidique de formule (I).

En outre, la présente invention a pour troisième objet l'utilisation d'une composition cosmétique comprenant ledit composé peptidique de formule (I) pour prévenir et/ou traiter les signes cutanés du vieillissement et du photo-vieillissement et améliorer la dégradation par le protéasome des protéines endommagées.

Enfin, la présente invention a pour quatrième objet un procédé de traitement cosmétique de la peau ou des phanères à traiter à l'aide de la composition comprenant ledit composé peptidique de formule(I).

La présente invention a pour objet un composé peptidique de formule générale suivante (I) :

R₁ - X₁ - Arg - Lys - Gly - X₂ - R₂

Dans laquelle,
X₁ représente une isoleucine, une leucine, une proline, une valine, ou est égal à zéro,
X₂ représente une cystéine, un acide glutamique, une glutamine, une histidine, une méthionine, une phénylalanine, une tyrosine, une thréonine, ou est égal à zéro,
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide amine C-terminal, substitué par un groupement protecteur pouvant être choisi parmi une chaîne alkyle de C₁ à C₂₀, ou un groupement NH2, NHY ou NYY avec Y représentant une chaîne alkyle de C₁ à C₄,
ladite séquence de formule générale (I) étant constituée de 3 à 5 résidus d'acides aminés,
ladite séquence de formule générale (I) pouvant comprendre des substitutions des acides aminés X₁ et X₂, par d'autres acides aminés chimiquement équivalents.

Le composé peptidique selon l'invention est caractérisé en ce qu'il permet d'augmenter l'activité du protéasome.

On entend par composé peptidique qui « permet d'augmenter l'activité du protéasome», tout peptide ou dérivé biologiquement actif capable d'augmenter l'activité du protéasome, soit par augmentation de la synthèse protéique des sous-unités du protéasome (par modulation directe ou indirecte de l'expression génique), soit par d'autres processus biologiques tels que la stabilisation des sous-unités constituant le protéasome ou encore la stabilisation des transcrits d'ARN messager.

Le composé peptidique selon l'invention est caractérisé en ce qu'il permet d'activer la dégradation par le protéasome des protéines endommagées. Par « protéines endommagées » on entend des protéines ayant subi des réactions d'oxydation dues aux espèces réactives de l'oxygène (radicaux libres), des protéines glyquées ou conjuguées avec des produits issus de la péroxydation lipidique etc.

Dans un premier mode de réalisation préféré, le composé peptidique est protégé par une acylation ou par une acétylation de l'extrémité amino-terminale et/ou de l'extrémité carboxy-terminale.

Dans un second mode de réalisation préféré, le composé peptidique correspond à une des formules suivantes :
(SEQ ID n° 1) Ile - Arg - Lys - Gly - NH₂
(SEQ ID n°2) Leu - Arg - Lys - Gly -Met- NH₂
(SEQ ID n°3) Pro - Arg - Lys - Gly -Thr - NH₂
(SEQ ID n°4) Val - Arg - Lys - Gly -Phe - NH₂
(SEQ ID n°5) Val - Arg - Lys - Gly - Tyr - NH₂
(SEQ ID n°6) Arg - Lys - Gly - NH₂

Préférentiellement, le composé peptidique selon l'invention correspond à la séquence ID n°2 c'est-à-dire Leu - Arg - Lys - Gly -Met- NH₂.

Dans un autre mode de réalisation préféré, le composé peptidique selon l'invention correspond à la séquence ID n°6 c'est-à-dire Arg - Lys - Gly - NH₂.

L'invention concerne aussi des formes homologues de ces séquences. Le terme « homologue » désigne, selon l'invention, toute séquence peptidique identique à au moins 50%, ou de préférence au moins 80%, et encore plus préférentiellement à au moins 90% de ladite séquence peptidique, choisie parmi les séquences SEQ ID n° 1 à SEQ ID n° 6. Par « séquence peptidique identique à au moins X% », on entend désigner un pourcentage d'identité entre les résidus d'acides aminés des deux séquences à comparer, obtenue après l'alignement optimal des deux séquences. L'alignement optimal est obtenu à l'aide d'algorithmes d'homologies locales tels que ceux utilisés par le logiciel informatique BLAST P disponible sur le site NCBI.

Le terme « homologue » peut également désigner un peptide qui diffère de la séquence d'un peptide de séquence SEQ ID n° 1 à SEQ ID n° 6 par la substitution d'acides aminés chimiquement équivalents, c'est-à-dire par la substitution d'un résidu par un autre possédant les mêmes caractéristiques. Ainsi, les substitutions classiques se font entre Ala, Val, Leu et Ile ; entre Ser et Thr ; entre les résidus acides Asp et Glu ; entre Asn et Gln ; et entre les résidus basiques Lys et Arg ; ou entre les résidus aromatiques Phe et Tyr.

Les acides aminés constituant le peptide selon l'invention peuvent être sous configuration Lévogyre c'est-à-dire L- et/ou Dextrogyre c'est-à-dire D-. Le peptide selon l'invention peut donc être sous forme L-, D- ou DL-.

Le terme « peptide » ou « composé peptidiques » désigne un enchaînement de deux ou plusieurs acides aminés liés entre eux par des liaisons peptidiques ou par des liaisons peptidiques modifiées.

Par « peptide » ou « composé peptidique », il faut entendre le peptide naturel ou synthétique de l'invention tel que décrit ci-dessus, ou au moins l'un de ses fragments, qu'il soit obtenu par protéolyse ou de manière synthétique, ou encore tout peptide naturel ou synthétique dont la séquence est totalement ou partiellement constituée par la séquence du peptide précédemment décrit.

De façon à améliorer la résistance à la dégradation, il peut être nécessaire d'utiliser une forme protégée du peptide selon l'invention. La forme de protection doit évidemment être une forme biologiquement compatible et doit être compatible avec une utilisation dans le domaine des cosmétiques ou de la pharmacie.

De nombreuses formes de protection biologiquement compatibles peuvent être envisagées. Elles sont bien connues de l'homme du métier comme, par exemple, l'acylation ou l'acétylation des extrémités amino et/ou carboxy-terminales. Ainsi, l'invention concerne une composition telle que définie précédemment, caractérisée par le fait que le peptide de séquence SEQ ID n°1 à SEQ ID n°6 est sous forme protégée de façon simple ou double. De préférence, on utilise, pour la protection de la fonction hydroxyle de l'extrémité carboxy-terminale, une amidation par un groupement NYY avec Y représentant une chaîne alkyle de C₁ à C₄, ou l'estérification par un groupement alkyle. Il est également possible de protéger les deux extrémités du peptide.

Le peptide de formule générale (I) selon l'invention peut être obtenu soit par synthèse chimique classique (en phase solide ou en phase homogène liquide), soit par synthèse enzymatique (Kullman et al., J. Biól. Chem. 1980, 225, 8234), à partir d'acides aminés constitutifs ou de leurs dérivés.

Le peptide selon l'invention peut être d'origine naturelle ou synthétique. Préférentiellement selon l'invention, le peptide est obtenu par synthèse chimique.

Enfin, le principe actif peut être un peptide unique, un mélange de peptides ou de dérivés peptidiques et/ou constitué de dérivés d'acides aminés.

Le composé peptidique selon l'invention peut être utilisé à titre de médicament.

Le second objet de la présente invention se rapporte à une composition cosmétique comprenant à titre de principe actif ledit composé peptidique de formule générale (I). De manière préférée, les compositions selon l'invention se présentent sous une forme adaptée à l'application par voie topique comprenant un milieu cosmétiquement acceptable. Par « cosmétiquement acceptable », on entend des milieux qui conviennent à une utilisation en contact avec la peau ou les phanères humains, sans risque de toxicité, d'incompatibilité, d'instabilité, de réponse allergique et autres. Préférentiellement, ledit composé peptidique est présent dans la composition à une concentration comprise entre 0,0005 et 500 ppm environ, et préférentiellement à une concentration comprise entre 0,01 et 5 ppm. Dans les compositions selon l'invention, le composé peptidique est solubilisé dans un ou plusieurs solvants comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux, le principe actif selon l'invention est solubilisé dans un vecteur cosmétique ou pharmaceutique comme les liposomes ou adsorbé sur des polymères organiques poudreux, des supports minéraux comme les talcs et bentonites, et plus généralement solubilisé dans, ou fixé sur, tout vecteur physiologiquement acceptable.

Les compositions destinées à être appliquées sur la peau peuvent se présenter sous forme de solution aqueuse ou hydroalcoolique, d'émulsion eau dans huile ou huile dans eau, de microémulsion, de gel aqueux ou anhydre, de sérum, ou encore de dispersion de vésicules, de patch, de crème, de spray, d'onguent, de pommade, de lotion, de colloïde, de solution, de suspension ou autres. Les compositions peuvent aussi être appliquées sur les phanères sous forme de shampoing, de teinture ou de mascara à appliquer au pinceau ou au peigne, en particulier sur les cils, les sourcils ou les cheveux, ou encore de soins pour les ongles tels que les vernis.

Dans un mode de réalisation particulier, la composition selon l'invention contient en outre, au moins un autre principe actif favorisant l'action dudit composé peptidique. On peut citer, de manière non limitative, les classes d'ingrédients suivantes : d'autres agents actifs peptidiques, des extraits de végétaux, des agents cicatrisants, anti-âge, anti-rides, apaisants, anti-radicalaire, anti-UV, des agents stimulant la synthèse de macromolécules dermiques ou le métabolisme énergétique, des agents hydratants, anti-bactériens, antifongiques, anti-inflammatoires, anesthésiques, des agents modulants la différenciation, la pigmentation ou la dépigmentation cutanée, des agents stimulants la pousse des ongles ou des cheveux.... Préférentiellement, on utilisera un agent présentant une activité dans le domaine des antirides, tel qu'un agent anti-radicalaire ou antioxydant, ou un agent stimulant la synthèse de macromolécules dermiques, ou encore un agent stimulant le métabolisme énergétique. Plus particulièrement, le principe actif est choisi parmi les vitamines, les phytostérols, les flavonoïdes, la DHEA et/ou un de ses précurseurs ou un de ses dérivés chimiques ou biologiques, un inhibiteur de métalloproteinase, ou un rétinoïde. En outre, des additifs tels que des agents épaississants, émulsifiants, humectants, émollients, des parfums, des antioxydants, des agents filmogènes, chélatants, séquestrants, conditionnants....peuvent être ajoutés à la composition.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, être compris entre 0,01 et 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés dans une proportion allant de 0,3 à 30 % en poids, par rapport au poids total de la composition.

Enfin, l'invention se rapporte à une composition comprenant le dit composé peptidique afin d'augmenter l'activité du protéasome et d'améliorer la dégradation par le protéasome des protéines endommagées.

Un troisième objet de l'invention se rapporte à l'utilisation d'une composition cosmétique comprenant ledit composé peptidique et un milieu cosmétiquement acceptable pour prévenir et/ou traiter les signes cutanés du vieillissement et du photo-vieillissement.

Les « signes cutanés du vieillissement » incluent, mais ne se limitent pas à, toutes les manifestations visibles sur la peau causées par le vieillissement. On entend en particulier les rides, les rides profondes et grossières, les ridules, les crevasses, le relâchement des tissus cutanés et sous-cutanés, la perte de l'élasticité cutanée et l'atonie, la perte de fermeté et de tonicité, et l'atrophie dermique. En outre, on entend aussi par « signes cutanés du vieillissement », les pores élargis, les imperfections, la décoloration, les taches de vieillesse, les kératoses, la perte de collagène, et autres changements du derme et de l'épiderme, mais également toutes modifications de l'aspect extérieur de la peau et des phanères dues au vieillissement comme, par exemple, les rugosités superficielles de la couche cornée, mais également toute modification interne de la peau qui ne se traduit pas systématiquement par un aspect extérieur modifié comme, par exemple, l'amincissement du derme. Par « photo-vieillissement » on entend le vieillissement prématuré de la peau causé par l'exposition prolongée et cumulative au soleil.

La présente invention se rapporte donc à l'utilisation d'une composition pour traiter ou prévenir les rides, les rides profondes et grossières, les ridules, les crevasses, le relâchement des tissus cutanés et sous-cutanés, la perte de l'élasticité cutanée et l'atonie, la perte de fermeté et de tonicité, et l'atrophie dermique. En outre, ladite composition selon l'invention permet d'activer le renouvellement cellulaire et de nettoyer en profondeur les cellules.

Un autre objet de l'invention concerne l'utilisation d'une composition selon l'invention pour protéger la peau contre les agressions dues aux rayonnements UV. Enfin, l'invention se rapporte à l'utilisation d'une composition comprenant le composé peptidique pour augmenter l'activité du protéasome et améliorer la dégradation par le protéasome des protéines endommagées.

Un dernier objet de la présente invention se rapporte à un procédé de traitement cosmétique caractérisé en ce que l'on applique topiquement sur la peau ou les phanères à traiter, une composition comprenant une quantité efficace de composé peptidique selon l'invention pour prévenir et/ou traiter les signes cutanés du vieillissement et du photo-vieillissement. En outre, ce procédé de traitement cosmétique est caractérisé en ce que la composition est appliquée avant le coucher de façon à nettoyer les cellules et la peau pendant le cycle de renouvellement cellulaire. En effet, durant la nuit, la peau privilégie les fonctions de renouvellement ainsi que les processus métaboliques de synthèse. Par conséquent, l'application de la composition telle que revendiquée, en respectant le rythme biologique de la peau permet d'obtenir un effet rajeunissant, stimulant le renouvellement cellulaire, et régénérant.

Les exemples suivants décrivent et démontrent l'efficacité de composés peptidiques tels que décrits selon l'invention mais ne doivent pas être interprétés comme une limitation de la présente invention.

### Exemple 1 : Validation du modèle des kératinocytes âgés : Immunoblotting des protéines ubiquitinylées et du protéasome 20S sur kératinocytes âgés

### Processus de vieillissement des kératinocytes en culture

Des cellules NHK, Normal Human Keratinocytes, sont isolées à partir de biopsies de peau humaine. Ces cellules sont maintenues en culture dans un milieu spécial kératinocyte-SFM (Invitrogen) à 37°C sous une atmosphère humide de 5 % CO₂. On obtient des cellules NHK vieillies en effectuant une série de sous-cultures répétées. Brièvement les cellules sont ensemencées dans des flasques de 75 cm², à densité faible et mises en culture de 5 à 10 jours entre 2 passages. Les cellules sont traitées ou non par le principe actif, par ajout de l'actif directement dans le milieu de culture 3 fois par semaine. Ces cellules sont ensuite maintenues dans ces conditions pendant 45 jours.

### Immunoblotting des protéines ubiquitinylées et du protéasome 20S sur kératinocytes âgées

Afin de valider notre modèle, il convenait tout d'abord de démontrer que sur des kératinocytes en culture vieillis expérimentalement, le taux d'expression des protéines ubiquitinylées, ainsi que le taux de protéasome 20S, conformément à la littérature, diminuaient au cours du temps. Le taux d'expression de ces protéines a été évalué par la technique de l'immunoblotting. La technique de l'immunoblotting (ou western blotting) est une méthode semi-quantitative qui permet d'apprécier le taux de protéines étudiées dans les cellules, c'est-à-dire le taux des protéines ubiquitinylées par un anticorps spécifiques de l'ubiquitine (DAKO), ainsi que le taux de protéasome 20S par un anticorps spécifique du protéasome (CALBIOCHEM).

### Protocole:

Des kératinocytes vieillis sont mis en culture dans des flasques de 75 cm² à 37°C dans une atmosphère humidifiée contenant 5 % de CO₂ pendant 10, 20, 30 et 40 jours. Les cellules ne sont pas traitées, le jour de l'expérience. Elles sont rincées puis détachées du support à l'aide d'un tampon d'extraction (20 mM TRIS, 150 mM NaCl, 10 mM EDTA, 0.2 % Triton X10) en présence d'un cocktail d'inhibiteurs de protéases (Sigma). Les protéines ainsi extraites sont centrifugées à 4°C à 10000 rpm pendant 10 minutes avant d'être dosées par le kit de dosage des protéines BCA (Pierce). Les lysats cellulaires sont mélangés à un tampon de dénaturation et soumis à une électrophorèse SDS PAGE. Le gel utilisé est un Nupage 4-12 % (Invitrogen). Les protéines sont enfin transférées sur une membrane de nitrocellulose (Pal corporation). Les membranes sont saturées dans du PBS-lait 5 %, 0,1% tween 20, 2 heures à température ambiante, puis incubées à 4°C toute la nuit avec un anticorps primaire.
Pour l'étude des protéines ubiquitinylées l'anticorps primaire est un anticorps de lapin anti-ubiquitin utilisé à la dilution de 1/500^{ème} (DAKO).
Pour l'étude du protéasome 20S l'anticorps primaire est un anticorps de souris anti-proteasome 20S utilisé à la dilution de 1/1000^{ème} (CALBIOCHEM).

L'incubation avec l'anticorps primaire est suivie d'une incubation avec un anticorps secondaire anti-lapin IgG-péroxydase (IMMUNOTECH) dilué au 1/1000^{ème} dans le cas des protéines ubiquitinylées, ou avec un anticorps secondaire anti-souris IgG-péroxydase (IMMUNOTECH) dilué au 1/1000^{ème} dans le cas du protéasome. La révélation est effectuée par un substrat chimioluminescent. L'évaluation des protéines exprimées dans les cellules est effectuée grâce au logiciel Chemiimager (Alpha Innotech Corporation USA). La quantité des protéines étudiées est exprimée en pourcentage d'intensité lumineuse comparée à la condition contrôle au temps 0 de l'expérience.

### Résultats :

**Immunoblotting des protéines ubiquitinylées sur des kératinocytes au cours du vieillissement :**

| Intensité (%) | Expérience 1 |
|---|---|
| Non traitées 10 jours | 100 % |
| Non traitées 20 jours | 71 % |
| Non traitées 30 jours | 64 % |
| Non traitées 50 jours | 56 % |

**Immunoblotting du protéasome 20S sur des kératinocytes au cours du vieillissement**

| Intensité (%) | Expérience 1 |
|---|---|
| Non traitées 10 jours | 100 % |
| Non traitées 20 jours | 94 % |
| Non traitées 30 jours | 47 % |
| Non traitées 50 jours | 54 % |

### Conclusion :

Nous avons donc démontré, dans notre système de vieillissement expérimental, que le taux de protéines ubiquitinylées diminuait au cours du temps et, proportionnellement au temps de culture, au bout de 40 jours nous enregistrons une diminution d'environ 56 % par rapport au temps 0. De la même manière nous avons démontré dans notre système de vieillissement expérimental, que le taux de protéasome 20S diminuait au cours du temps et, proportionnellement au temps de culture, au bout de 40 jours nous enregistrons une diminution d'environ 54 % par rapport au temps 0.

Ces 2 expériences nous permettent de conclure que le vieillissement expérimental mis en place au laboratoire est un bon modèle d'étude du vieillissement sur les cellules en culture. Ce modèle pourra donc être utilisé dans les expériences ultérieures pour démontrer l'activité et l'efficacité de notre actif.

### Exemple 2 : Mise en évidence de l'effet activateur de l'actif sur la mesure de l'activité enzymatigue du proteasome 20S sur des kératinocytes vieillis maintenus en culture.

Des kératinocytes vieillis expérimentalement maintenus en culture pendant 15 jours, sont traités avec 1% de notre peptide SEQ ID n°6. On étudie alors les activités enzymatiques du protéasome 20S. En effet le protéasome 20S est la sous-unité responsable de l'hydrolyse enzymatique. Trois activités enzymatiques peuvent être étudiées: l'activité trypsine-like, chimotrypsine-like et peptidylglutamyl-peptide hydrolase (PGPH). Nous nous proposons d'étudier ces activités par un dosage enzymatique spécifique de chaque activité.

### Protocole:

Les kératinocytes vieillis sont maintenus en culture pendant 15 jours. Le traitement des cellules est effectué par ajout d'une solution à 1 % de notre peptide SEQ ID n°6 directement dans le milieu, renouvelé 3 fois par semaine pendant le temps de l'expérience.

Le dosage de chaque activité a été mis en place en utilisant des substrats spécifiques marqués par un composé fluorescent: le 7-amido-4-méthylcoumarine (AMC). Après clivage, l'AMC dont la longueur d'onde d'excitation est de 350nm, la lecture de la fluorescence est effectuée à 440nm. L'intensité de la fluorescence est proportionnelle à la quantité de fluorochrome obtenue et en conséquence, cette quantité est proportionnelle à la quantité de substrat hydrolysé.

Le peptide synthétique Boc-Leu-Arg-Arg-AMC est spécifique de l'activité trypsique. Le peptide synthétique Suc-Leu-Leu-Val-Try-AMC est spécifique de l'activité chymotrypsique.
Le peptide synthétique Z-Leu-Leu-Glu-AMC est spécifique de l'activité peptidylglutamyl-peptide hydrolase.
Ces peptides ont été fournis et marqués par SIGMA ALDRICH, Saint-Louis, MI, USA.

Les cellules sont détachées du support dans un tampon d'extraction. Par la suite, elles sont soniquées pendant 1 minute à 4°C, puis centrifugées à 15000 g pendant 30 minutes à 4°C. Le dosage des protéines est réalisé par le kit BCA (Pierce). Après incubation du lysat cellulaire avec le substrat synthétique spécifique de l'activité étudiée, la fluorescence est lue au spectrophotomètre Synergy (BIOTEK, Vermont, USA) à 440nm.

### Résultats:

Nous constatons que pour les 3 activités étudiées, le peptide SEQ ID n°6 a permis d'augmenter l'activité enzymatique du protéasome 20S. L'activité trypsine-like est augmentée de 155.3 % lors du traitement par l'actif, l'activité chymotrypsin-like est augmentée de 130 % et on enregistre une augmentation de 144.6 % pour l'activité peptidylglutamyl-peptide hydrolase.

### Conclusions :

Le peptide SEQ ID n°6 utilisé à 1 % sur des kératinocytes vieillis expérimentalement en culture permet d'augmenter les activités enzymatiques spécifiques du protéasomes 20S. L'expérience a été réalisée plusieurs fois, et un test statistique (test statistique t-Student) a pu être réalisé. L'augmentation des activités est significative pour l'étude de l'activité trypsine-like, chymotrypsin-like (p=0.033 et p=0.0477 respectivement), et hautement sigriificative pour l'activité peptidylglutamyl-peptide hydrolase (p=0.00053).

### Exemple 3: Mise en évidence de l'effet activateur de l'actif sur la prolifération des kératinocytes vieillis en culture

Une étude de l'effet activateur du peptide SEQ ID n°2 a été réalisée en évaluant l'expression de la protéine marqueur de la prolifération cellulaire, sur des kératinocytes vieillis expérimentalement en culture. L'analyse s'est effectuée après 20 jours de culture.

### Immuno-dosage :

Des études *in vitro* de kératinocytes vieillis expérimentalement en culture pendant 20 jours, ont permis de mettre en évidence par immunomarquage, l'expression de la protéine Ki67 qui est un marqueur de la prolifération. Les kératinocytes sont mis en culture pendant 20 jours dans un incubateur à CO₂ à 37°C sous atmosphère humide. Les cellules sont traitées directement par ajout de 1 % de peptide directement dans le milieu. Des cellules non traitées mais vieillies de la même manière sont utilisées comme cellules témoins. Pour les besoins de l'expérience les cellules sont ensemencées dans des labteck 8 puits, maintenues en culture et traitées dans ce même support jusqu'au jour du marquage. Le jour de l'immunomarquage, les cellules sont rincées à l'aide de tampon phosphate, puis fixées au méthanol froid avant le marquage.
L'immunomarquage s'effectue par un anticorps lapin anti-Ki67 (Abcam) dilué au 1/100^{ième} et un anticorps secondaire au 1/1000^{ème} couplé à un fluorochrome Alexa fluor singe anti-lapin (Molecular probes).
Les lames sont alors montées dans un milieu adéquat et observées au microscope à épifluorescence (Nikon Eclipse E600).

### Résultats / Conclusions :

Les résultats obtenus montrent que l'intensité des cellules marquées dans les conditions témoins est moins importante que dans les conditions traitées par l'actif

L'actif a permis d'augmenter la protéine Ki67 dans les cellules vieillies et traitées comparé aux cellules non traitées. Le marqueur de prolifération Ki67 a permis de mettre en évidence une prolifération des kératinocytes vieillis lorsqu'ils sont traités par l'actif.

### Exemple 4: Mise en évidence de l'effet anti-vieillissement de l'actif sur des kératinocytes vieillis en culture

Une étude de l'effet anti-vieillissement de l'actif a été réalisée en évaluant l'expression de la protéine bêta-galactosidase sur des kératinocytes vieillis expérimentalement en culture. En effet, le marquage de la bêta-galactosidase est connu pour être présent dans les cellules sénescentes alors qu'on ne trouve pas d'activité galactosidase dans les cellules pré-sénescentes, quiescentes ou immortelles.

### Protocole :

Des kératinocytes vieillis expérimentalement dans des labteck 8 puits sont mis en culture et sont maintenus pendant 20 jours en présence ou non de 1 % de peptide de SEQ ID n°6. Le traitement est effectué 3 fois par semaine par ajout direct dans le milieu.
Des cellules non traitées sont maintenues en culture pendant le même temps de l'expérience et serviront de contrôle. Le jour du marquage, les cellules sont rincées, fixées dans un mélange 2 % glutaraldéhyde - 2 % formaldéhyde pendant 3 minutes. Les cellules sont ensuite rincées et on applique 300 µl de 5-bromo-4-chloro-3 indolyl β-D-galactosidase appelé communément X-gal (substrat de la bêta-galactosidase). L'incubation s'effectue pendant 24 heures dans l'incubateur à CO₂, puis les cellules sont rincées et la labteck est rapidement montée dans un milieu adéquat. L'observation s'effectue au microscope à transmission. Le principe est simple : lorsque les cellules sont sénescentes et contiennent la bêta-galactosidase, le substrat X-gal est clivé en un produit bleu insoluble. L'activité bêta-galactosidase est mise en évidence par une coloration des cellules en bleues. Plus il y a de cellules bleues, plus le nombre de cellules sénescentes est élevé.

### Résultats / conclusions :

Nous constatons qu'en présence de l'actif, l'activité bêta-galactosidase est fortement réduite dans les cellules traitées comparée aux cellules non traitées.

Par conséquent, l'actif permet de mettre en évidence un effet anti-vieillissement sur des kératinocytes en culture vieillis expérimentalement pendant 20 jours de culture.

### Exemple 5 : Evaluation de la carbonylation des protéines sur des fibroblastes traités par l'actif et soumis à des rayonnements ultraviolets (UVB)

### Protocole :

Des fibroblastes humains normaux en culture sont ensemencés dans des boites de diamètres 100. Lorsque les cellules ont atteint 70 % de confluence, les cellules sont traitées 48h avec le peptide de séquence ID n°6 dilué à 1 % dans le milieu. Les cellules sont soumises à l'irradiation UVB à 100mj/cm², puis remises 48h supplémentaires en présence de l'actif. Des boîtes contrôle avec des cellules non traitées par l'actif mais irradiées servent de témoin. Les cellules sont rincées puis détachées du support à l'aide d'un tampon d'extraction adéquat. Les protéines ainsi extraites, sont centrifugées à 4°C à 10000 rpm pendant 10 minutes avant d'être dosées par le kit de dosage des protéines BCA (Pierce). La carbonylation des protéines est réalisée par un test basé sur l'immunodétection des groupements carbonylés préalablement dérivés par le 2,4-dinitrophénylhydrazine (DNP) (SIGMA).

Selon la réaction :

Protéine-C=O + H₂N-NH-₂.₄DNP → Protéine-C=N-NH₂.₄-DNP + H₂O

Brièvement, 15 µl de l'échantillon sont mis à réagir avec 45 µl de DNP pendant 45 minutes à température ambiante. Ensuite, 5µl du mélange est dilué dans 1 ml de phosphate buffer saline et 200 µl de cette dilution sont mis dans une plaque 96 puits over night à 4°C en présence de 150 µl de BSA (fraction V).

Après 3 lavages en tampon phosphate saline (PBS), l'anticorps biotinylé de lapin anti-dinitrophenyl (CALBIOCHEM) est diluée au 1/5000^{ème} dans du tampon 0.1 % sérum albumine en présence de 0.1 % Tween 20 et incubé dans les microplaques 1 heure à 37°C. Après 3 lavages, on incube le complexe streptavidine-péroxydase (DAKO) dilué au 1/3000^{ème} dans du tampon 0.1 % sérum albumine en présence de 0.1 % Tween 20 et incubé dans les microplaques pendant 1 heure à température ambiante. Après 3 lavages, la révélation est effectuée grâce à 200 µl de tétramethylbenzidine (TMB, SIGMA) 25 minutes à température ambiante. Ensuite, l'ajout de 100 µl d'acide sulfurique à 2,5 M permet de stopper la réaction. La lecture de la DO s'effectue à 490 nm. Afin de convertir la DO obtenue en groupements carbonylés présents dans les échantillons, une courbe étalon a été établie en faisant varier des proportions de 0 à 100 % de BSA oxydée.

### Résultats :

En présence d'UVB les cellules non traitées sont fortement carbonylées et augmentent de 110 % par rapport aux cellules non irradiées et non traitées. En présence de l'actif à 1 % le taux de carbonylation est diminué de 34 %. L'expérience a été réalisée plusieurs fois et un test statistique (test statistique t Student) a pu être réalisé. La diminution de la carbonylation est significative et p= 0.0298.

En conclusion, l'actif permet de protéger les cellules contre les effets néfastes des UV c'est-à-dire contre leurs effets oxydatifs. L'actif permet de diminuer de plus de 34 % l'oxydation des protéines.

## Revendications

1. Composé peptidique de formule générale suivante (I) :
R₁ - X₁ - Arg - Lys - Gly - X₂ - R₂
Dans laquelle,
X₁ représente une isoleucine, une leucine, une proline, une valine, ou est égal à zéro,
X₂ représente une cystéine, un acide glutamique, une glutamine, une histidine, une méthionine, une phénylalanine, une tyrosine, une thréonine, ou est égal à zéro,
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, substitué par un groupement protecteur pouvant être choisi parmi une chaîne alkyle de C₁ à C₂₀, ou un groupement NH2, NHY ou NYY avec Y représentant une chaîne alkyle de C₁ à C₄,
ladite séquence de formule générale (I) étant constituée de 3 à 5 résidus d'acides aminés, ladite séquence de formule générale (1) pouvant comprendre des substitutions des acides aminés X₁ et X₂, par d'autres acides aminés chimiquement équivalents.

2. Composé peptidique selon la revendication 1, **caractérisé en ce qu'**il correspond à une des formules suivantes :
(SEQ ID n°1) Ile - Arg - Lys - Gly - NH₂
(SEQ ID n°2) Leu - Arg - Lys - Gly -Met- NH₂
(SEQ ID n°3) Pro - Arg - Lys - Gly -Thr - NH₂.
(SEQ ID n°4) Val - Arg - Lys - Gly -Phe - NH₂
(SEQ ID n°5) Val - Arg - Lys - Gly - Tyr - NH₂
(SEQ ID n°6) Arg - Lys - Gly - NH₂

3. Composé peptidique selon l'une des revendications 1 ou 2 **caractérisé en ce qu'**il est utilisé à titre de médicament.

4. Composition cosmétique comprenant à titre de principe actif ledit composé peptidique selon l'une des revendications 1 ou 2.

5. Composition cosmétique selon la revendication 4, **caractérisée en ce qu'**elle se présente sous une forme adaptée à l'application par voie topique comprenant un milieu cosmétiquement acceptable.

6. Composition selon l'une des revendications 4 ou 5, **caractérisée en ce que** ledit composé peptidique est présent dans la composition à une concentration comprise entre 0,0005 est 500 ppm environ, et préférentiellement à une concentration comprise entre 0,01 et 5 ppm.

7. Composition selon l'une quelconque des revendications 4 à 6, **caractérisée en ce qu'**elle contient en outre, au moins un autre principe actif favorisant l'action dudit composé peptidique, tel qu'un agent anti-radicalaire ou antioxydant, ou un agent stimulant la synthèse de macromolécules dermiques, ou encore un agent stimulant le métabolisme énergétique.

8. Composition selon la revendication 7, **caractérisée en ce que** ledit principe actif est choisi parmi les vitamines, les phytostérols, les flavonoïdes, la DHEA et/ou un de ses précurseurs ou un de ses dérivés chimiques ou biologiques, un inhibiteur de métalloproteinase, ou un rétinoïde.

9. Utilisation d'une composition cosmétique comprenant ledit composé peptidique tel que défini dans l'une des revendications 1 ou 2 et un milieu cosmétiquement acceptable pour prévenir et/ou traiter les signes cutanés du vieillissement et du photo-vieillissement.

10. Utilisation d'une composition selon la revendication 9, **caractérisée en ce que** par signe du vieillissement on entend les rides, les rides profondes et grossières, les ridules, les crevasses, le relâchement des tissus cutanés et sous-cutanés, la perte de l'élasticité cutanée et l'atonie, la perte de fermeté et de tonicité, et l'atrophie dermique.

11. Utilisation selon la revendication 9, **caractérisée en ce que** ladite composition permet d'activer le renouvellement cellulaire et de nettoyer en profondeur les cellules.

12. Utilisation selon la revendication 9, **caractérisée en ce que** ladite composition permet de protéger la peau contre les agressions dues aux rayonnements UV.

13. Utilisation d'une composition cosmétique selon la revendication 9 pour augmenter l'activité du protéasome et améliorer la dégradation par le protéasome des protéines endommagées.

14. Procédé de traitement cosmétique **caractérisé en ce que** l'on applique topiquement sur la peau ou les phanères à traiter, une composition comprenant une quantité efficace de composé peptidique tel que défini dans l'une des revendications 1 ou 2, pour prévenir et/ou traiter les signes cutanés du vieillissement et du photo-vieillissement.

15. Procédé de traitement cosmétique, tel que défini dans la revendication précédente, **caractérisé en ce que** la composition est appliquée avant le coucher de façon à nettoyer la peau pendant le cycle de renouvellement cellulaire.

## Patentansprüche

1. Peptidverbindung der folgenden allgemeinen Formel (I):
R₁-X₁-Arg-Lys-Gly-X₂-R₂,
wobei
X₁ für ein Isoleucin, ein Leucin, ein Prolin oder ein Valin steht oder gleich null ist,
X₂ für ein Cystein, eine Glutaminsäure, ein Glutamin, ein Histidin, ein Methionin, ein Phenylalanin, ein Tyrosin oder ein Threonin steht oder gleich null ist,
R₁ für die primäre Aminofunktion der N-terminalen Aminosäure steht, frei oder durch eine Schutzgruppe substituiert, die aus einer Acetylgruppe, einer Benzoylgruppe, einer Tosylgruppe oder einer Benzyloxycarbonylgruppe ausgewählt sein kann,
R₂ für die Hydroxylgruppe der Carboxylfunktion der C-terminalen Aminosäure steht, substituiert durch eine Schutzgruppe, die aus einer C₁- bis C₂₀-Alkylkette oder einer NH₂-, NHY- oder NYY-Gruppe, in der Y für eine C₁-bis C₄-Alkylkette steht, ausgewählt sein kann,
wobei die besagte Sequenz der allgemeinen Formel (I) aus 3 bis 5 Aminosäureresten besteht,
wobei die besagte Sequenz der allgemeinen Formel (I) Substitutionen der Aminosäuren X₁ und X₂ durch andere chemisch äquivalente Aminosäuren umfassen kann.

2. Peptidverbindung nach Anspruch 1, **dadurch gekennzeichnet, dass** sie einer der folgenden Formeln entspricht:
(SEQ ID Nr. 1) Ile-Arg-Lys-Gly-NH₂
(SEQ ID Nr. 2) Leu-Arg-Lys-Gly-Met-NH₂
(SEQ ID Nr. 3) Pro-Arg-Lys-Gly-Thr-NH₂
(SEQ ID Nr. 4) Val-Arg-Lys-Gly-Phe-NH₂
(SEQ ID Nr. 5) Val-Arg-Lys-Gly-Tyr-NH₂
(SEQ ID Nr. 6) Arg-Lys-Gly-NH₂.

3. Peptidverbindung nach einem der Ansprüche 1 oder 2, **dadurch gekennzeichnet, dass** sie als Arzneimittel verwendet wird.

4. Kosmetische Zusammensetzung, die die besagte Peptidverbindung nach einem der Ansprüche 1 oder 2 als Wirkstoff umfasst.

5. Kosmetische Zusammensetzung nach Anspruch 4, **dadurch gekennzeichnet, dass** sie in einer Form dargereicht wird, die auf eine topische Anwendung angepasst ist und die ein kosmetisch akzeptables Medium umfasst.

6. Zusammensetzung nach einem der Ansprüche 4 oder 5, **dadurch gekennzeichnet, dass** die besagte Peptidverbindung in der Zusammensetzung in einer Konzentration zwischen 0,0005 bis ungefähr 500 ppm und vorzugsweise in einer Konzentration zwischen 0,01 und 5 ppm vorliegt.

7. Zusammensetzung nach einem der Ansprüche 4 bis 6, **dadurch gekennzeichnet, dass** sie außerdem mindestens einen anderen Wirkstoff enthält, der die Wirkung der besagten Peptidverbindung begünstigt, wie ein Radikalfänger oder ein Antioxidans oder ein Mittel, das die Synthese von dermalen Makromolekülen stimuliert, oder auch ein Mittel, das den Energiestoffwechsel stimuliert.

8. Zusammensetzung nach Anspruch 7, **dadurch gekennzeichnet, dass** der besagte Wirkstoff aus Vitaminen, Phytosterinen, Flavonoiden, DHEA und/oder einem seiner Vorläufer oder einem seiner chemischen oder biologischen Derivate, einem Metalloproteinase-Hemmer oder einem Retinoid ausgewählt ist.

9. Verwendung einer kosmetischen Zusammensetzung, die die wie in einem der Ansprüche 1 oder 2 definierte besagte Peptidverbindung und ein kosmetisch akzeptables Medium umfasst, zum Verhindern und/oder Behandeln von Zeichen der Alterung und der Lichtalterung der Haut.

10. Verwendung einer Zusammensetzung nach Anspruch 9, **dadurch gekennzeichnet, dass** unter Zeichen der Alterung Falten, tiefe und grobe Falten, Fältchen, Risse, Erschlaffung der Haut- und Unterhautgewebe, Verlust der Hautelastizität und Atonie, Verlust der Festigkeit und des Tonus und dermale Atrophie verstanden werden.

11. Verwendung nach Anspruch 9, **dadurch gekennzeichnet, dass** die besagte Zusammensetzung das Aktivieren der Zellerneuerung und das Tiefenreinigen der Zellen ermöglicht.

12. Verwendung nach Anspruch 9, **dadurch gekennzeichnet, dass** die besagte Zusammensetzung das Schützen der Haut vor Angriffen aufgrund von UV-Strahlen ermöglicht.

13. Verwendung einer kosmetischen Zusammensetzung nach Anspruch 9 zum Steigern der Aktivität des Proteasoms und Verbessern des Abbaus von geschädigten Proteinen durch das Proteasom.

14. Verfahren zur kosmetischen Behandlung, **dadurch gekennzeichnet, dass** eine Zusammensetzung, die eine wirksame Menge der wie in einem der Ansprüche 1 oder 2 definierten Peptidverbindung umfasst, zum Verhindern und/oder Behandeln von Zeichen der Alterung und der Lichtalterung der Haut topisch auf die zu behandelnde Haut oder die zu behandelnden Hautanhangsgebilde angewendet wird.

15. Verfahren zur kosmetischen Behandlung, wie in dem vorherigen Anspruch definiert, **dadurch gekennzeichnet, dass** die Zusammensetzung vor dem Schlafengehen angewendet wird, um die Haut während des Zellerneuerungszyklus zu reinigen.

## Claims

1. Peptide compound having the following general formula (I):
R₁-X₁-Arg-Lys-Gly-X₂-R₂
Wherein,
X₁ represents an isoleucine, a leucine, a proline, a valine, or is equal to zero,
X₂ represents a cysteine, a glutamic acid, a glutamine, a histidine, a methionine, a phenylalanine, a tyrosine, a threonine, or is equal to zero,
R₁ represents the primary amine function of the N-terminal amino acid, free or substituted by a protecting group that may be chosen from among an acetyl group, a benzoyl group, a tosyl group or a benzyloxycarbonyl group,
R₂ represents the hydroxyl group of the carboxyl function of the C-terminal amino acid, free or substituted by a protecting group that may be chosen from among a C₁ to C₂₀ alkyl chain, or an NH2, NHY or NYY group where Y represents a C₁ to C₄ alkyl chain,
said sequence having the general formula (I) consisting of 3 to 5 amino acid residues,
said sequence having the general formula (I) optionally comprising substitutions of the amino acids X₁ and X₂, by other chemically equivalent amino acids.

2. Peptide compound according to claim 1, **characterised in that** it corresponds to one of the following formulae:
(SEQ ID No. 1) Ile-Arg-Lys-Gly-NH₂
(SEQ ID No. 2) Leu-Arg-Lys-Gly-Met-NH₂
(SEQ ID No. 3) Pro-Arg-Lys-Gly-Thr-NH₂
(SEQ ID No. 4) Val-Arg-Lys-Gly-Phe-NH₂
(SEQ ID No. 5) Val-Arg-Lys-Gly-Tyr-NH₂
(SEQ ID No. 6) Arg-Lys-Gly-NH₂

3. Peptide compound according to any one of claims 1 or 2 **characterised in that** it is used as a medicine.

4. Cosmetic composition comprising, as an active ingredient, said peptide compound according to any one of claims 1 or 2.

5. Cosmetic composition according to claim 4, **characterised in that** it is presented in a form suitable for topical application comprising a cosmetically acceptable medium.

6. Composition according to any one of claims 4 or 5, **characterised in that** said peptide compound is present in the composition at a concentration between approximately 0.0005 and 500 ppm, and preferentially at a concentration between 0.01 and 5 ppm.

7. Composition according to any one of claims 4 to 6, **characterised in that** it further contains at least one further active ingredient promoting the action of said peptide compound, such as an anti-radical or anti-oxidant agent or an agent stimulating the synthesis of dermal macromolecules, or else an agent stimulating the energy metabolism.

8. Composition according to claim 7, **characterised in that** said active ingredient is chosen from vitamins, plant sterols, flavonoids, DHEA and/or any of the precursors thereof or any of the chemical or biological derivatives thereof, a metalloproteinaise inhibitor, or a retinoid.

9. Use of a cosmetic composition comprising said peptide compound as defined in any one of claims 1 or 2 and a cosmetically acceptable medium for preventing and/or treating the skin signs of ageing and photo-ageing.

10. Use of a composition according to claim 9, **characterised in that** the term sign of ageing denotes wrinkles, deep and coarse wrinkles, lines, cracks, slackening of cutaneous and subcutaneous tissue, loss of skin elasticity and atony, loss of firmness and tone, and dermal atrophy.

11. Use according to claim 9, **characterised in that** said composition is suitable for activating cell renewal and cleansing cells in-depth.

12. Use according to claim 9, **characterised in that** said composition is suitable for protecting the skin against damage caused by UV radiation.

13. Use of a cosmetic composition according to claim 9 for increasing proteasome activity and enhancing degradation of damaged proteins by the proteasome.

14. Cosmetic treatment method **characterised in that** a composition comprising an effective quantity of peptide compound as defined in any one of claims 1 or 2 is applied topically onto the skin or skin appendages to be treated, for preventing and/or treating the skin signs of ageing and photo-ageing.

15. Cosmetic treatment method, as defined in the preceding claim, **characterised in that** the composition is applied before going to sleep so as to cleanse the skin during the cell renewal cycle.
